**Europäisches Patentamt**

**European Patent Office**

**Office européen des brevets**

(19)

(11) Veröffentlichungsnummer: **0 029 156**
**B2**

(12)

# NEUE EUROPÄISCHE PATENTSCHRIFT

(45) Veröffentlichungstag der neuen Patentschrift:
**30.03.88**

(51) Int. Cl.⁴: **C 07 C 87/60, C 07 C 85/26**

(21) Anmeldenummer: **80106745.5**

(22) Anmeldetag: **03.11.80**

(54) Verfahren zur Herstellung von farbstabilem o-Chloranilin.

(30) Priorität: **14.11.79 DE 2945868**

(43) Veröffentlichungstag der Anmeldung:
**27.05.81 Patentblatt 81/21**

(45) Bekanntmachung des Hinweises auf die Patenterteilung:
**20.07.83 Patentblatt 83/29**

(45) Bekanntmachung des Hinweises auf die Entscheidung
über den Einspruch:
**30.03.88 Patentblatt 88/13**

(84) Benannte Vertragsstaaten:
**BE DE FR GB IT**

(56) Entgegenhaltungen:
**DE - C - 942 992**
**US - A - 2 447 615**
**US - A - 2 911 340**
**US - A - 2 927 136**
**US - A - 3 154 583**

**Ullmanns Encyklopädie der technischen Chemie, 4.
Auflage, Band 7, Seite 571
Anzen Kogatu 21 (1982) Heft 2 S. 107-114 "Gefahren bei
der Destillation chlorierter aromatischer Amine".**

(73) Patentinhaber: **BAYER AG, Konzernverwaltung RP
Patentabteilung, D-5090 Leverkusen 1 Bayerwerk (DE)**

(72) Erfinder: **Kritzler, Helmuth, Dr., Zum Hahnenberg 74,
D-5068 Odenthal (DE)**
Erfinder: **Böhm, Walter, Dr.,
Elisabeth-Langgässer-Strasse 34, D-5090 Leverkusen
(DE)**
Erfinder: **Kappler, Ulrich, Dr., Bamberger Strasse 16,
D-5090 Leverkusen (DE)**
Erfinder: **Winkelmann, Hans Dieter, Dr. c/o Mobay
Chemical, Corporation Dyestuff Division Bushy Park
PO 10288, Charleston South Carolina 29411 (US)**

## Beschreibung

Verfahren zur Herstellung von farbstabilem o-Chloranilin.

Gegenstand der vorliegenden Erfindung ist ein Verfahren zur Herstellung von farbstabilem o-Chloranilin.

Es ist bekannt, dass die Herstellung von farblosen aromatischen Aminen Schwierigkeiten bereitet (DE-AS 1 518 107). Selbst solche aromatischen Amine, die unmittelbar nach der Herstellung farblos erscheinen, verfärben sich oft nach kurzer Zeit und sind damit sehr häufig für die Weiterverarbeitung, z.B. zu Farbstoffen, nicht mehr geeignet, da sie die Eigenschaften der Produkte verändern.

Es wurde deshalb nach Verbindungen gesucht, die in sehr geringen Mengen den aromatischen Aminen zugesetzt werden können und die die Verfärbung der aromatischen Amine beim Lagern verhindern oder zumindest für längere Zeit unterdrücken sollen.

In der Literatur sind eine Reihe von Farbstabilisatoren beschrieben, die eine Verfärbung bei aromatischen Aminen verhindern sollen.

Beispielsweise werden als geeignete Farbstabilisatoren erwähnt: Hydrazin oder Hydrazinverbindungen (DE-AS 1 518 107, US-PS 1 973 724), das Reaktionsprodukt aus $P_2S_5$ mit Polyalkylphenol (US-PS 2 510 849), Kohlenstoffdisulfid (US-PS 2 434 651), 2-Alkylalkoxyphenole (US-PS 2 442 457), Xylylbiguanide (US-PS 2 469 745) und Thiocarbanilide (US-PS 2 493 544).

Es hat sich aber gezeigt, dass halogenierte aromatische Amine, die mit Stabilisatoren versetzt worden waren, bei der Weiterverarbeitung zu gefärbten Folgeprodukten führten. So eignet sich z.B. ein mit Hydrazinhydrat stabilisiertes o-Chloranilin nicht für die Herstellung von 3,3'-Dichlor-4,4'-diaminodiphenylmethan, das einen Härter für giessfähige Polyuräthanelastomere darstellt.

Es wurde nun ein Verfahren zur Herstellung von farbstabilem o-Chloranilin gefunden, das dadurch gekennzeichnet ist, dass man das in üblicher Weise hergestellte o-Chloranilin in Apparaturen aus Edelstählen destilliert und gegebenenfalls das destillierte o-Chloranilin in Behältern aus dem genannten Material aufbewahrt und transportiert.

Bevorzugt werden Apparaturen aus Cr-Ni-Mo-Stählen verwendet. Die Zusammensetzung und die Bezeichnung der Cr-Ni-Mo-Stähle sind z.T. aufgeführt in Benennungssystem verschiedener Länder, z.B. in dem deutschen System DIN 17 440 sowie dem amerikanischen System ASTM-A 167-74, ASTM-A 479-75 und ASTM-A 666-72.

Für das erfindungsgemässe Verfahren eignen sich besonders Apparaturen aus Cr-Ni-Mo-Stählen mit folgender Zusammensetzung (in Gew.%): Kohlenstoff: ≦0,10; Silicium: 1,0; Mangan: 2,0; Chrom: 16,5 bis 18,5; Molybdän: 2,0 bis 2,5; Nikkel: 10,5 bis 13,5 und Titan (Werkstoff Nr. 1.4571; DIN 17 440) oder der Zusammensetzung: Kohlenstoff: ≧0,04; Mangan: ≦2,0; Chrom: 16,5 bis 18,5; Molybdän: 4,0 bis 5,0; Nickel: 12,5 bis 14,5 und Stickstoff: 0,10 bis 0,20 (Werkstoff-Nr. 1.4439; Stahl-Eisen-Werkstoffblatt 400, S. 8, 4. Auflage, Dezember 1973).

Das erfindungsgemässe Verfahren kann bei Normaldruck oder bei Unterdruck durchgeführt werden. Bevorzugt destilliert man bei Unterdruck. Es wird im allgemeinen bei Drücken von 1 mbar bis Normaldruck, bevorzugt bei 5 bis 35 mbar destilliert.

Die Destillationstemperatur hängt vom jeweils angelegten Druck ab und beträgt üblicherweise 70 bis 250°C, bevorzugt 80 bis 100°C.

Es hat sich als zweckmässig erwiesen, o-Chloranilin unmittelbar nach dessen Herstellung zu destillieren.

Zur längeren Aufbewahrung des erfindungsgemäss destillierten o-Chloranilins ist es vorteilhaft, dieses in Behältern aus den zuvor erwähnten Materialien zu lagern.

Auch für den Transport ist es empfehlenswert, das o-Chloranilin in Behältern aus den genannten Materialien zu transportieren.

Dadurch wird ein späteres Verfärben des o-Chloranilins verhindert.

Das nach dem erfindungsgemässen Verfahren behandelte o-Chloranilin ist ausserordentlich farbbeständig, d.h. es behält seine Farblosigkeit über Monate, wenn es in Behältern aus den genannten Materialien gelagert und transportiert wird.

Es kann selbst bei längerer Lagerung ohne vorherige Reinigung und/oder Destillation direkt z.B. zur Weiterverarbeitung für Farbstoffe oder Polyuräthanhärter verwendet werden.

Es ist ausserordentlich überraschend, dass die Farbbeständigkeit des o-Chloranilins durch einfache Destillation erreicht werden kann.

Ein Zusatz von speziellen, eventuell für die Weiterverarbeitung des o-Chloranilins störenden Farbstabilisatoren ist nicht mehr erforderlich.

Beispiel

Ein aus o-Nitrochlorbenzol durch katalytische Hydrierung mit Platin auf Kohle in Toluol nach den üblichen Methoden hergestelltes o-Chloranilin wurde nach Abtrennen des Katalysators und Abdestillieren des Toluols in einer Apparatur aus Cr-Ni-Mo-Stahl, der 16,5 bis 18,5% Chrom; ≦0,10% Kohlenstoff; 1,0% Silicium; 2,0% Mangan; 2,0 bis 2,5% Molybdän; 10,5 bis 13,5% Nickel und ≧5% Titan enthielt, bei einer Temperatur von 78°C und einem Druck von 5 mbar destilliert.

Das frisch destillierte o-Chloranilin hatte eine Farbzahl von 40 Hazen (Bestimmung der Hazen-Farbzahl gemäss DIN 53 409) und wurde in Behältern aus verschiedenen Materialien über 5 Monate bei 20°C gelagert (Tabelle 1).

Tabelle 1

| Material des Behälters | Unmittelbar nach der Destillation | Farbzahl nach Harzen | | | | |
|---|---|---|---|---|---|---|
| | | nach 1 Monat | nach 2 Monaten | nach 3 Monaten | nach 4 Monaten | nach 5 Monaten |
| Eisen (Vergleich) | 40 | 160 | 260 | >500[1] | >500 | >500 |
| Cr-Ni-Mo-Stahl Werkstoff-Nr. 1.4571 (DIN 17 440) | 40 | 40 | 40 | 40 | 40 | 40 |
| Cr-Ni-Mo-Stahl Werkstoff-Nr. 1.4439 (Stahl-Eisen-Werkstoffblatt 400, S.8) | 40 | 40 | 40 | 40 | 40 | 40 |

[1] Die Farbskala nach Harzen geht nur bis 500.

**Patentansprüche**

1) Verfahren zur Herstellung von farbstabilem o-Chloranilin, dadurch gekennzeichnet, dass man das in üblicher Weise hergestellte o-Chloranilin in Apparaturen aus Edelstählen destilliert und gegebenenfalls das destillierte o-Chloranilin in Behältern aus dem genannten Material aufbewahrt und transportiert.

2) Verfahren nach Anspruch 1, dadurch gekennzeichnet, dass man das o-Chloranilin in Apparaturen aus Cr-Ni-Mo-Stählen destilliert.

**Claims**

1. Process for the preparation of o-chloroaniline which is stable in colour, characterised in that o-chloroaniline prepared in the customary manner is distilled in apparatuses made of stainless steels and, if appropriate, the distilled o-chloraniline is stored and transported in containers made of the stated material.

2. Process according to Claim 1, characterised in that the o-chloroaniline is distilled in apparatuses made of Cr/Ni/Mo steels.

**Revendications**

1. Procédé de fabrication d'o-chloraniline de coloration stable, caractérisé en ce qu'on distille l'o-chloraniline, fabriquée de la manière usuelle, dans des appareillages en aciers spéciaux et en ce que l'on conserve et transporte éventuellement l'o-chloraniline distillée dans des récipients en les matériaux cités.

2. Procédé selon la revendication 1, caractérisé en ce qu'on distille l'o-chloraniline dans des appareillages en aciers Cr-Ni-Mo.